# FASCICULE DE BREVET EUROPEEN

(11) **EP 0 792 636 B1**
(45) Date de publication et mention de la délivrance du brevet: **01.04.1998**
(21) Numéro de dépôt: 97400125.7
(22) Date de dépôt: 21.01.1997
(51) Int. Cl.: A61K 7/48, A61K 7/032

(54) **Composition cosmétique comprenant un polymère filmogène et des esters de sucre**
Kosmetische Zusammensetzung enthaltend ein filmbildendes Polymer und Zuckerester
Cosmetic composition comprising a filmforming polymer and sugar esters

(30) Priorité: 29.02.1996 FR 9602561
(43) Date de publication de la demande: 03.09.1997
(73) Titulaire: L'OREAL, 75008 Paris (FR)
(72) Inventeur: Felardos, Christian, 94550 Chevilly Larue (FR); Aygat-Cano, Christin, 77310 Saint Fargeau Ponthierry (FR); Collin, Nathalie, 92330 Sceaux (FR)
(74) Mandataire: Dodin, Catherine

(56) Documents cités:
- EP-A- 0 042 157
- EP-A- 0 647 443
- WO-A-94/17775

## Description

L'invention a trait à de nouvelles compositions cosmétiques, en particulier des compositions de mascara ayant des propriétés de maquillage très performantes alliées à une très grande douceur. L'invention concerne plus particulièrement de nouvelles compositions de mascara à base d'au moins un polymère filmogène et d'esters de sucres.

Il existe sur le marché de nombreuses formulations de mascara; toutefois, le consommateur attend toujours une amélioration des propriétés de ces mascaras que sont l'allongement du cil, le recourbement du cil, une meilleure adhésion de la composition sur le cil, une meilleure tolérance par le cil.

On connaît par le document US-5,389,363 des compositions de mascara comprenant un polymère polyester dispersible dans l'eau, ce mascara possédant de bonnes propriétés d'adhérence et d'allongement du cil. Toutefois, cette propriété d'allongement n'est obtenue que par l'action conjuguée de ce polyester et d'un autre polymère synthétique oléosoluble, les deux polymères étant introduits respectivement dans un gel et dans une émulsion huile dans eau, les deux phases étant préparées extemporanément puis mélangées pour donner la composition de mascara. La composition comme le procédé de préparation de ce mascara présentent l'inconvénient d'être complexes.

On connaît par ailleurs, d'après le document US-5,053,221, des compositions de mascara comprenant un polymère polyester dispersible dans l'eau, ne s'écaillant pas, léger, doux et allongeant pour les cils, démaquillable à l'eau. Ces compositions ont la particularité de ne pas comprendre de cires. En revanche, elles comprennent des particules microsphériques qui contribuent à la rapidité du séchage. Ces compositions peuvent en outre comprendre comme émulsifiant du sesquistéarate de méthylglucose et du sesquistéarate de méthyl gluceth-20. Ces compositions de mascara, qui ne comprennent pas de cires, présentent l'inconvénient de donner un maquillage trop naturel, peu allongeant, peu recourbant, qui n'adhère pas bien au cil.

L'homme du métier connaît par ailleurs la très bonne tolérance par la peau et par les cheveux des esters de sucre lorsqu'ils sont utilisés comme émulsionnants. Toutefois si l'on prépare une composition à partir d'ingrédients classiques des compositions de mascara (cires, pigments, gélifiants, filmogènes traditionnels comme l'hydroxyéthylcellulose, agents de séchage comme la silice) et comprenant un polymère polyester filmogène dispersible dans l'eau et des esters d'acides gras du glucose, polyoxyéthylénés ou non, on obtient des résultats assez décevants qui ne permettent pas de prédire un intérêt particulier de cette catégorie de produits dans des compositions de mascara.

Aussi, c'est avec étonnement que la demanderesse a découvert de nouvelles compositions de mascara à base d'au moins un polymère filmogène et d'esters de sucres, qui, par comparaison avec les compositions de l'art antérieur, permettent un meilleur allongement et un meilleur recourbement du cil, une meilleure adhésion de la composition sur le cil, alliés à une très bonne tolérance par le cil et par l'oeil.

Les compositions selon l'invention sont caractérisées par le fait qu'elles comprennent:
- au moins une cire,
- au moins un polymère de type polyester, filmogène anionique, et
- au moins un ester d'acide gras et de saccharose.

Avantageusement, les compositions selon l'invention sont caractérisées par le fait qu'elles comprennent, en poids par rapport au poids total de la composition :
- 5 à 30% d'au moins une cire,
- 0,1 à 25% d'au moins un polymère de type polyester, filmogène et anionique, et
- 0,5 à 20% d'au moins un ester d'acide gras et de saccharose.

Dans la présente description, les pourcentages de produits dans les compositions selon l'invention sont donnés en masse par rapport à la masse totale de la composition. La masse de polymère dans la composition est comptée en matière sèche.

L'utilisation d'esters de sucre selon l'invention permet d'obtenir une composition dont la consistance permet une application facile et une bonne adhésion du mascara. Les formules sont très bien tolérées, même chez les utilisatrices ayant les yeux sensibles, étant donné que les esters de sucre employés sont non-ioniques, donc présentant une innocuité améliorée. En outre, après le démaquillage des cils, ceux-ci conservent une apparence de douceur et de souplesse. De plus l'utilisation d'esters de sucre selon l'invention permet de stabiliser les émulsions, notamment les émulsions cire-dans-eau, ne comprenant pas d'autre émulsionnant que lesdits esters.

Selon un mode préféré de réalisation de l'invention, le taux d'estérification du saccharose dans l'ester d'acide gras et de saccharose est supérieur ou égal à deux.
De façon préférentielle, l'ester d'acide gras et de saccharose est choisi parmi les produits répondant à la formule (I) : 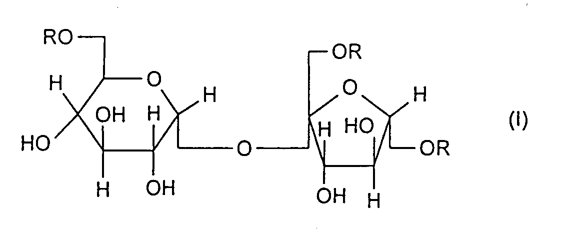 dans laquelle les substituants R, identiques ou différents, représentent un proton ou un groupement : 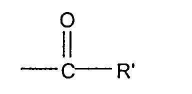 avec R' représentant un groupement alkyle, linéaire ou ramifié, saturé ou insaturé en C12-C22, l'un au moins des substituants R étant distinct du proton. Avantageusement, on choisit R' parmi les groupements alkyle, linéaires ou ramifiés, saturés ou insaturés en C16-C18.

Selon un mode préféré de réalisation de l'invention, les compositions selon l'invention comprennent en outre au moins un ester de monosaccharide et/ou d'alkyl monosaccharide. Ce monosaccharide peut être choisi par exemple parmi les pentoses, parmi lesquels on peut citer le D-ribose, le D-arabinose, le D-xylose, le D-lyxose, le D-ribulose, le D-xylulose ; les hexoses, comme par exemple le D-allose, le D-altrose, le D-glucose, le D-mannose, le D-gulose, le D-idose, le D-galactose, le D-talose, le D-fructose, le D-sorbose et le D-tagatose ; les heptoses comme le D-mannoheptulose, le D-sedoheptulose.

De préférence, le monosaccharide est un alkyl glucose.
Avantageusement, les compositions selon l'invention comprennent en outre jusqu'à 15% en poids d'au moins un ester d'alkyl glucose.
Selon un mode préféré de réalisation de l'invention on choisit l'ester d'alkyl glucose parmi les esters gras de C1-C6 alkyl glucose polyoxyéthylénés.
Préférentiellement, l'ester d'alkyl glucose est choisi parmi les produits répondant à la formule (II) : 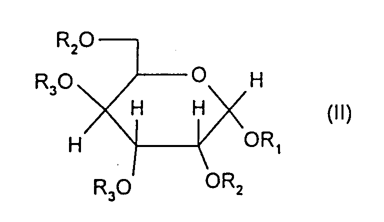 dans laquelle R₁ représente un atome d'hydrogène ou un groupement alkyle, linéaire ou ramifié, saturé ou insaturé en C1-C6, les groupements R₂, identiques ou différents, représentent un proton ou un groupement :

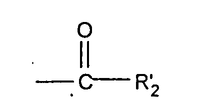

R'₂ représentant un groupement alkyle, linéaire ou ramifié, saturé ou insaturé en C12-C22, l'un au moins des groupements R₂ étant distinct du proton, et les groupements R₃, identiques ou différents, représentent un atome d'hydrogène ou un groupement -(CH₂-CH₂O)ₙ-H, l'un au moins des groupements R3 étant distinct du proton, avec n allant de 50 à 200.

De façon préférentielle, les compositions selon l'invention comprennent un mélange d'esters de sucre constitué de 30 à 100% d'au moins un ester de saccharose selon la formule (I) et de 0 à 70% d'au moins un ester de glucose selon la formule (II), en poids par rapport au poids total de la composition.
De façon encore plus préférentielle, les compositions selon l'invention comprennent un mélange d'esters de sucre constitué de 60 à 80% d'au moins un ester de saccharose selon la formule (I) et de 20 à 40% d'au moins un ester de glucose selon la formule (II). La quantité d'esters de sucre présents dans la composition selon l'invention est de préférence comprise entre 1 et 20% en poids.

Le polymère filmogène utilisé dans le cadre de l'invention est de type polyester, et peut comprendre en outre des séquences de type amide, amine, uréthanne et/ou des chaînes grasses. De préférence, le polymère filmogène est anionique et est dispersable dans l'eau. Il peut toutefois être solubilisé dans le milieu.

Les polymères filmogènes utilisables dans la présente invention sont choisis préférentiellement parmi les polymères polyester et/ou polyesteramide anioniques dispersables dans l'eau, comprenant des monomères portant une fonction -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut citer en particulier les polymères décrits dans les documents US-3,734,874 ; US-4,233,196 ; US-4,304,901.

Avantageusement, on utilise dans la présente invention des polymères polyesters filmogènes à base d'au moins un acide dicarboxylique, d'au moins un diol et d'au moins un monomère aromatique bifonctionnel portant en outre un groupement - SO₃M tel que décrit ci-dessus.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide sébarique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbo-rane dicarboxylique, l'acide diglycolique, l'acide thiodipropionique, l'acide 4,4'-sulfonyldibenzoïque, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement, l'acide phtalique, l'acide isophtalique, l'acide téréphtalique.
Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products. On peut également utiliser dans les compositions objet de la présente invention les polymères commercialisés par la société Rhône-Poulenc sous le nom de marque Polycare. De tels polymères polyester peuvent être utilisés seuls ou en mélanges.

Le polymère filmogène est utilisé en quantités qui permettent d'obtenir une bonne adhésion de la composition sur le cil, un allongement un gainage et une courbure qui donnent une impression d'ouverture du regard, les cils étant bien séparés. De façon préférentielle, les compositions selon l'invention comprennent une quantité de polymère filmogène allant de 0,1 à 25% en poids par rapport au poids total de la composition. Avantageusement, les compositions qui font l'objet de la présente invention comprennent une quantité allant de 1 à 10% en poids par rapport au poids total de la composition de polymère filmogène.

De façon préférentielle, les compositions selon la présente invention comprennent en outre au moins un co-émulsionnant choisi de préférence parmi les alcools gras, les acides gras et les esters gras de glycérol. Les chaînes grasses des alcools gras, des acides gras et des esters gras de glycérol sont habituellement d'une longueur allant de 12 à 22 atomes de carbone. On peut citer comme co-émulsionnant utilisable dans la présente invention : l'alcool stéarylique, l'alcool cétylique, le stéarate de glycéryle, l'acide stéarique. Les co-émulsionnants sont introduits dans les compositions selon l'invention en quantités allant de 0 à 10% en masse par rapport à la masse totale de l'invention, et préférentiellement de 1 à 4%.

Les cires peuvent être choisies parmi les cires animales et végétales, comme par exemple la cire de Carnauba, la cire de candelilla, la cire d'abeille, la cire de baleine, la cire de riz, les cires microcristallines, les lanolines ; avantageusement les cires sont présentes dans les compositions objet de l'invention en quantités allant de 5 à 30%, préférentiellement de 10 à 25% en poids.

Les compositions selon l'invention comprennent en outre des composés bien connus de l'homme du métier pour la préparation de compositions de mascara, et parmi lesquels on peut citer plus particulièrement : (a) l'eau, en quantités comprises entre 30 et 80% ; (b) des gélifiants, comme par exemple l'hydroxyéthyl cellulose, habituellement utilisés en quantités comprises entre 0 et 3% ; (c) des pigments, comme par exemple des oxydes métalliques en quantités allant de 0,5 à 20% ; (d) des émollients, habituellement utilisés en quantités comprises entre 1 et 10% ; (e) des conservateurs comme par exemple l'imidazolinyl urée, le méthylparaben, le propylparaben ; (f) des huiles volatiles, qui s'évaporeront au contact du cil, mais dont la présence dans la composition cosmétique est utile car elles facilitent l'étalement de la composition lors de l'application ; (g) d'autres corps gras, comme par exemple des mélanges de gomme ou de résine de silicone avec une huile volatile ; (h) des charges comme par exemple de la silice du kaolin, du talc, de la poudre de soie, utilisés habituellement en quantités comprises entre 0 et 15%; (i) des polymères filmogènes hydrophiles non-ioniques, en une quantité allant de 0 à 3% en poids.

Les compositions selon l'invention sont habituellement sous la forme d'émulsions cire dans eau ; elles peuvent également être sous la forme de dispersions aqueuses, de dispersions huileuses, d'émulsions eau dans huile, d'émulsions huile dans eau, d'émulsions eau dans cire et de gels.

Les compositions selon l'invention peuvent trouver une application en tant que produit de maquillage tel que mascara, rouge à lèvres, fond de teint, eye-liner; produit de coiffage ou de soin pour les cheveux; et/ou produit de soin de la peau tel que crème de soin ou produit solaire.

L'invention est illustrée plus en détail dans les exemples suivants.

Dans les compositions décrites ci-dessous, les composés utilisés sont :
a) comme polymère filmogène :
   - copolymères obtenus par condensation de di-éthylèneglycol, cyclohexane di-méthanol, acide isophtalique, acide sulfoisophtalique :
      - P1=Eastman AQ 55S,
      - P2=Eastman AQ 38S,
      - P3=Eastman AQ 48 ultra polymère, commercialisés par la société Eastman,
   - autre anionique, polyméthacrylate de sodium : -P4=Darvan 7 (commercialisé par la société Van Der Bilt),
   - autre : -P5=Ganex WP660 (commercialisé par la société ISP), copolymère polyvinylpyrrolidone/1-triacontène.
b) esters de sucre : tableau I

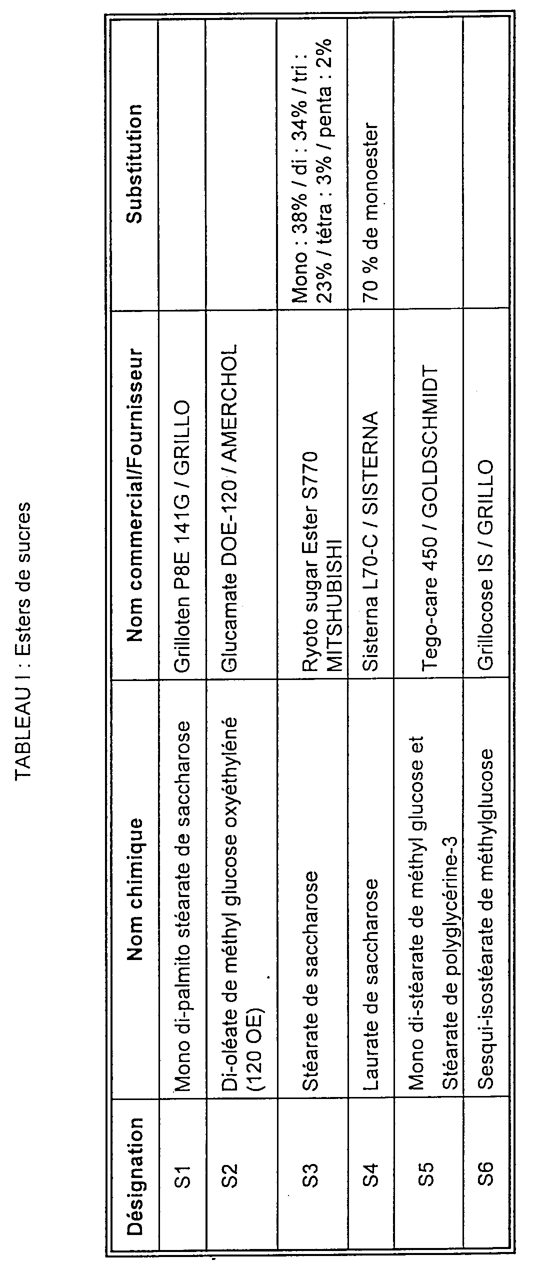

### Exemples 1 à 15 :

Des formules de mascara ont été préparées en formant un mélange homogène à partir de :
- - mélange de cires animales et végétales: 13,5%
- - oxyde de fer noir: 7%
- - polymère filmogène: p%
- - esters de sucre: s%
- - hydroxy-éthyl cellulose: 0,9%
- - huile de silicone: 0,15%
- - gomme de silicone (poids moléculaire 10⁵): 0,14%
- - cyclopentadiméthylsiloxane: 0,86%
- - alcool stéarylique à 98%: 2,25%
- - conservateur: 0,7%
- - eau déminéralisée stérilisée: qsp 100%
en faisant varier la nature et la quantité (p, s) du polymère filmogène et des esters de sucre.

Dans l'exemple 10, le polymère est remplacé par de l'eau.
Dans l'exemple 11, on remplace les esters de sucre par un mélange de substitution désigné MS et qui est constitué de 7,25% d'acide stéarique et de 3,75% de triéthanolamine, en poids par rapport au poids total de la composition.

Les qualités des mascaras sont notées de 1 à 5 suivant le barème :
- dispersion :: 1=très bien dispersé ; 5=dispersion grossière,
- fluidité :: 1=très fluide ; 5=très épais,
- adhérence :: 1=très bonne adhérence ; 5=peu adhérent,
- gainage :: 1=très gainant ; 5=très peu gainant,
- séchage :: 1=séchage très rapide ; 5=séchage très lent,
- allongement :: 1=très allongeant ; 5=très peu allongeant,
- courbure :: 1=très recourbant ; 5=très peu recourbant.
La viscosité est évaluée en Pa.s.

Les résultats des essais réalisés sont résumés dans le tableau II ci-après.

Les exemples 1 à 5, 8, 14-15 font partie de l'invention.

Les exemples 6-7 et 9-13 sont comparatifs.

### Exemple 16 :

On prépare des compositions de mascara selon une formule qui se distingue de celle donnée à l'exemple 1 par l'absence d'alcool stéarylique (0%) et éventuellement une quantité plus importante d'hydroxy-éthyl cellulose (0,9 à 2%).
Les compositions obtenues se présentent sous la forme d'émulsions fines très bien dispersées, ayant un pH aux environs de 7,5 et présentant une bonne adhérence sur le cil. Ces compositions, qui allongent et recourbent très bien le cil, donnent un maquillage très satisfaisant.

### Exemple 17 :

On prépare une composition de mascara selon une formule qui se distingue de celle donnée à l'exemple 1 par le remplacement des esters de sucre S1 et S2 par un mélange de 2% de sequistéarate de méthylglucose et de 6% de sequistéarate de méthylglucose oxyéthyléné, en masse par rapport à la masse totale de la composition. On obtient une formule ayant un pH de 5,75, trop acide pour les yeux. Cette formule doit donc être neutralisée par l'addition de 0,04% de soude. L'émulsion obtenue n'est pas très fine, elle est très fluide et n'adhère pas bien sur le cil. En outre, elle sèche très lentement. Le maquillage obtenu est trop naturel.

### Exemple 18 :

On prépare une composition de mascara selon une formule qui se distingue de celle donnée à l'exemple 1 par le remplacement des esters de sucre S1 et S2 par 8% de di-oléate de méthyl glucose non oxyéthyléné. La composition obtenue est trop liquide pour être utilisée comme mascara.

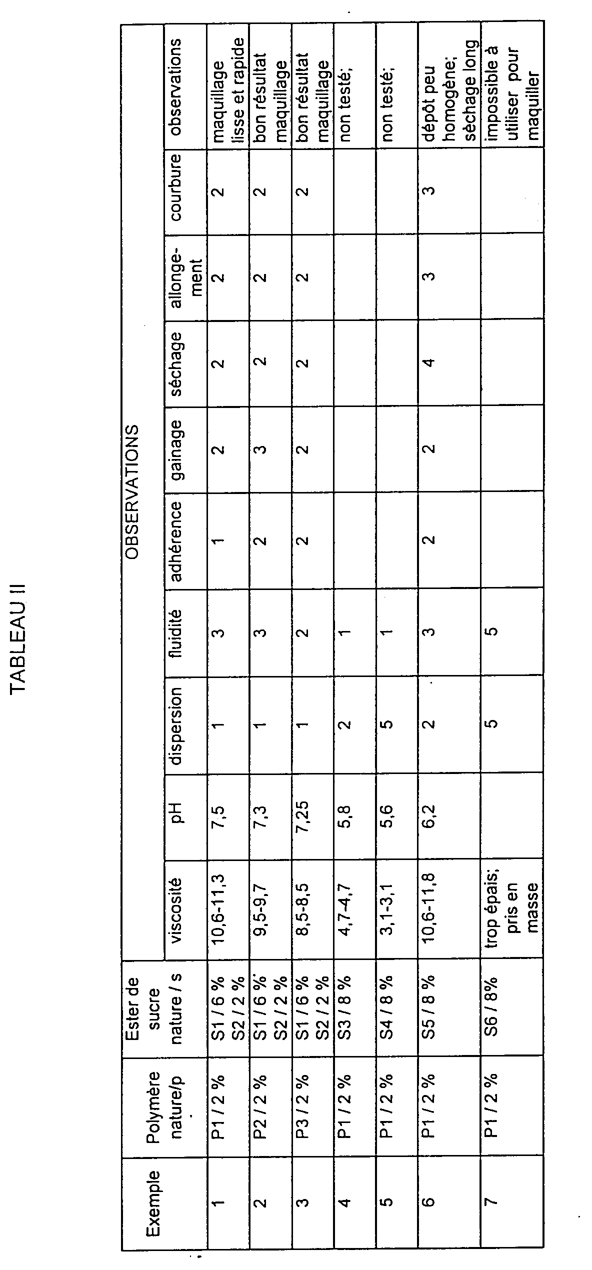

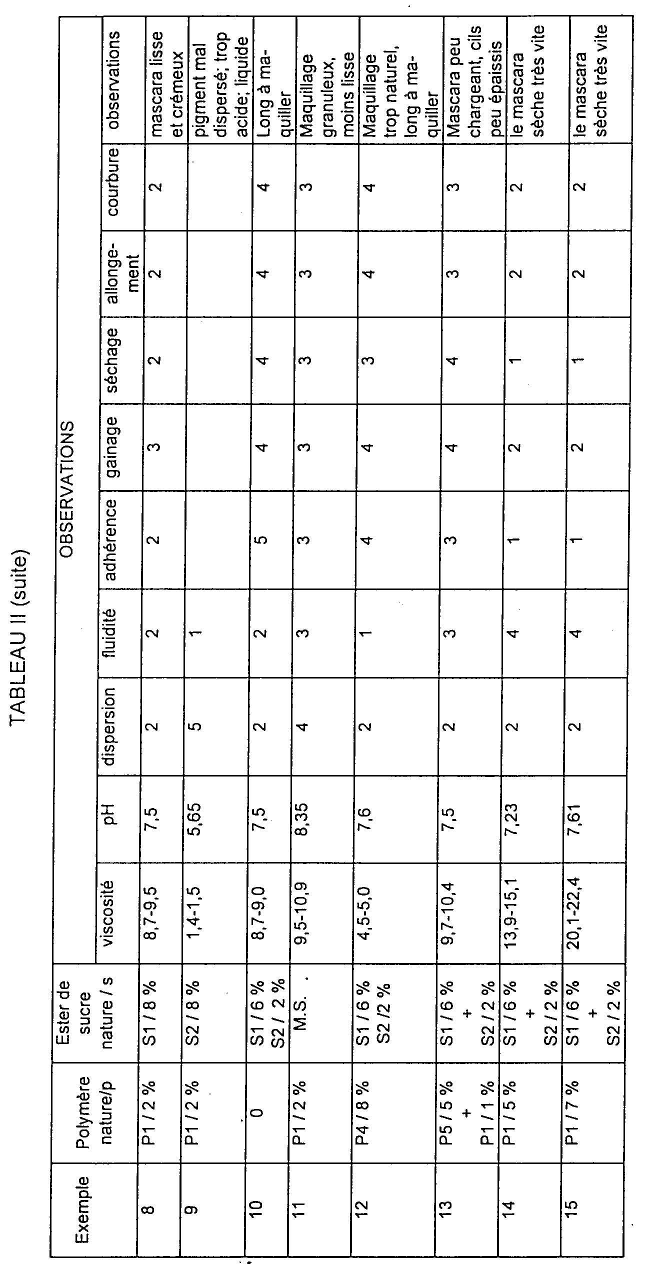

## Revendications

1. Composition cosmétique, caractérisée en ce qu'elle comprend :
- au moins une cire,
- au moins un polymère de type polyester, filmogène et anionique, et
- au moins un ester d'acide gras et de saccharose.

2. Composition selon la revendication 1, caractérisée en ce qu'elle comprend, en poids par rapport au poids total de la composition :
- 5 à 30% d'au moins une cire,
- 0,1 à 25% d'au moins un polymère de type polyester, filmogène et anionique, et
- 0,5 à 20% d'au moins un ester d'acide gras et de saccharose.

3. Composition selon l'une quelconque des revendications précédentes, caractérisée en ce que le taux d'estérification du saccharose dans l'ester d'acide gras et de saccharose est supérieur ou égal à deux.

4. Composition selon l'une quelconque des revendications précédentes, caractérisée en ce que l'ester d'acide gras et de saccharose est choisi parmi les produits répondant à la formule (I) : 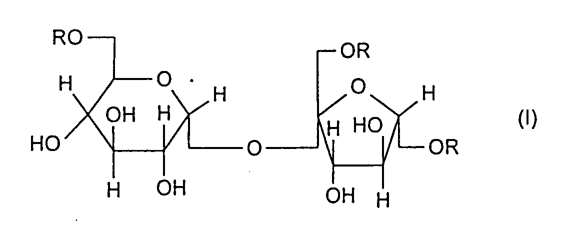 dans laquelle les substituants R, identiques ou différents, représentent un proton ou un groupement : 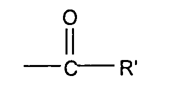 R' représentant un groupement alkyle, linéaire ou ramifié, saturé ou insaturé en C12-C22, l'un au moins des substituants R étant distinct du proton.

5. Composition selon l'une quelconque des revendications précédentes, caractérisée en ce qu'elle comprend en outre au moins un ester de monosaccharide et/ou d'alkyl monosaccharide.

6. Composition selon la revendication 5, caractérisée en ce que l'ester de monosaccharide et/ou d'alkyl monosaccharide est un ester d'alkyl glucose.

7. Composition selon la revendication 6, caractérisée en ce que l'ester d'alkyl glucose est choisi parmi les esters gras de C1-C6 alkyl glucose polyoxyéthylénés.

8. Composition selon l'une quelconque des revendications 6 à 7, caractérisée en ce que l'ester d'alkyl glucose est choisi parmi les produits répondant à la formule (II) : 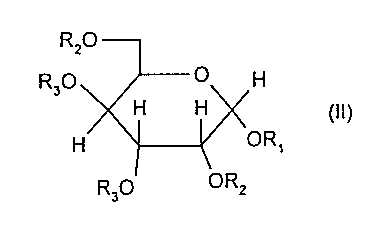 dans laquelle R₁ représente un atome d'hydrogène ou un groupement alkyle, linéaire ou ramifié, saturé ou insaturé en C1-C6, les groupements R₂, identiques ou différents, représentent un proton ou un groupement : 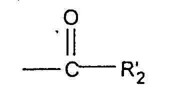 R'₂ représentant un groupement alkyle, linéaire ou ramifié, saturé ou insaturé en C12-C22, l'un au moins des groupements R₂ étant distinct du proton, et les groupements R₃, identiques ou différents, représentent un atome d'hydrogène ou un groupement -(CH₂-CH₂O)ₙ-H, l'un au moins des groupements R₃ étant distinct du proton, avec n allant de 50 à 200.

9. Composition selon l'une quelconque des revendications 6 à 8, caractérisée en ce qu'elle comprend jusqu'à 15% en poids d'au moins un ester d'alkyl glucose.

10. Composition selon l'une quelconque des revendications précédentes, caractérisée en ce qu'elle comprend un mélange d'esters de sucre constitué de 30 à 100% d'au moins un ester de saccharose selon la formule (I) et de 0 à 70% d'au moins un ester de glucose selon la formule (II), en poids par rapport au poids total de la composition.

11. Composition selon l'une quelconque des revendications précédentes, caractérisée en ce qu'elle comprend un mélange d'esters de sucre constitué de 60 à 80% d'au moins un ester de saccharose selon la formule (I) et de 20 à 40% d'au moins un ester de glucose selon la formule (II).

12. Composition selon l'une quelconque des revendications précédentes, caractérisée en ce que le polymère filmogène de type polyester comprend en outre des séquences de type amine, amide, uréthanne et/ou des chaînes grasses.

13. Composition selon l'une quelconque des revendications précédentes, caractérisée en ce que le polymère filmogène de type polyester est dispersable dans l'eau.

14. Composition selon l'une quelconque des revendications précédentes, caractérisée en ce que le polymère filmogène est choisi parmi les polymères polyester et/ou polyesteramide anioniques dispersables dans l'eau, comprenant des monomères portant une fonction -SO₃M, M représentant un atome d'hydrogène, un ion ammonium NH₄⁺ ou un ion métallique.

15. Composition selon l'une quelconque des revendications précédentes, caractérisée en ce que le polyester filmogène est à base d'au moins un acide dicarboxylique, d'au moins un diol et d'au moins un monomère aromatique bifonctionnel portant en outre un groupement -SO₃M.

16. Composition selon la revendication 15, caractérisée en ce que l'acide dicarboxylique est choisi parmi l'acide phtalique, l'acide isophtalique, l'acide téréphtalique.

17. Composition selon l'une quelconque des revendications 15 à 16, caractérisée en ce que le diol est choisi parmi : l'éthylène glycol, le diéthylène glycol, le triéthylène glycol, le 1,3-propanediol, le cyclohexane diméthanol, le 4-butanediol.

18. Composition selon l'une quelconque des revendications 15 à 17, caractérisée en ce que le monomère aromatique bifonctionnel portant en outre un groupement -SO₃M est choisi parmi: l'acide sulfoisophtalique, l'acide sulfotéréphtalique, l'acide sulfophtalique, l'acide 4-sulfonaphtalène-2,7-dicarboxylique.

19. Composition selon l'une quelconque des revendications précédentes, caractérisée en ce que le polymère filmogène est à base d'isophtalate/sulfoisophtalate.

20. Composition selon l'une quelconque des revendications précédentes, caractérisée en ce que le polymère filmogène est obtenu par condensation de di-éthylèneglycol, cyclohexane di-méthanol, acide isophtalique, acide sulfoisophtalique.

21. Composition selon l'une quelconque des revendications précédentes, caractérisée en ce qu'elle comprend une quantité de polymère filmogène allant de 0,1 à 25% en poids par rapport au poids total de la composition.

22. Composition selon la revendication 21, caractérisée en ce qu'elle comprend une quantité de polymère filmogène allant de 1 à 10% en poids par rapport au poids total de la composition.

23. Composition selon l'une quelconque des revendications précédentes, caractérisée en ce qu'elle comprend en outre au moins un co-émulsionnant.

24. Composition selon la revendication 23, caractérisée en ce que le co-émulsionnant est choisi parmi les alcools gras, les acides gras et les esters gras de glycérol.

25. Composition selon l'une quelconque des revendications précédentes, caractérisée en ce qu'elle comprend une quantité de cire allant de 5 à 30% en poids par rapport au poids total de la composition.

26. Composition selon l'une quelconque des revendications précédentes, caractérisée en ce qu'elle est sous la forme d'une émulsion cire-dans-eau, d'une dispersion aqueuse, d'une dispersion huileuse, d'une émulsion eau-dans-huile, d'une émulsion huile-dans-eau, d'une émulsion eau-dans-cire ou d'un gel.

27. Composition selon l'une quelconque des revendications précédentes, caractérisée en ce qu'elle est sous la forme d'un produit de maquillage tel que mascara, rouge à lèvres, fond de teint, eye-liner; d'un produit de coiffage ou de soin pour les cheveux; ou d'un produit de soin de la peau tel que crème de soin ou produit solaire.

28. Utilisation d'une composition selon l'une quelconque des revendications précédentes, caractérisée en ce qu'elle est utilisée pour préparer un mascara.

29. Utilisation d'un ester d'acide gras et de saccharose, dans une composition cosmétique comprenant au moins une cire et au moins un polymère de type polyester, filmogène et anionique, en tant qu'émulsionnant.

30. Utilisation d'un ester d'acide gras et de saccharose, dans une composition de mascara comprenant au moins une cire et au moins un polymère de type polyester, filmogène et anionique, dans le but d'améliorer l'allongement du cil, le recourbement du cil et/ou l'adhésion du mascara sur le cil.

31. Utilisation selon l'une des revendications 28 à 30, dans une composition de mascara destinée aux yeux sensibles.

## Claims

1. Cosmetic composition characterized in that it includes:
- at least one wax,
- at least one film-forming and anionic polymer of polyester type, and
- at least one ester of fatty acid and of sucrose.

2. Composition according to claim 1, characterized in that it includes, by weight in relation to the total weight of the composition:
- 5 to 30 % of at least one wax,
- 0.1 to 25 % of at least one film-forming and anionic polymer of polyester type, and
- 0.5 to 20 % of at least one ester of fatty acid and of sucrose.

3. Composition according to either of the preceding claims, characterized in that the degree of esterification of the sucrose in the ester of fatty acid and of sucrose is higher than or equal to two.

4. Composition according to any one of the preceding claims, characterized in that the ester of fatty acid and of sucrose is chosen from the products corresponding to the formula (I): 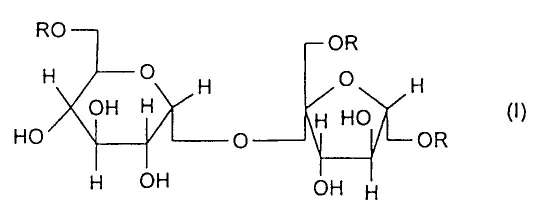 in which the substituents R, which are identical or different, denote a proton or a group: 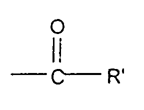 R' denoting a C₁₂-C₂₂ saturated or unsaturated, linear or branched alkyl group, at least one of the substituents R being other than a proton.

5. Composition according to any one of the preceding claims, characterized in that it additionally includes at least one ester of monosaccharide and/or alkyl monosaccharide.

6. Composition according to claim 5, characterized in that the ester of monosaccharide and/or alkyl monosaccharide is an alkyl glucose ester.

7. Composition according to claim 6, characterized in that the alkylglucose ester is chosen from the polyoxyethylenated fatty esters of (C₁-C₆-alkyl)glucose.

8. Composition according to either of claims 6 to 7, characterized in that the alkylglucose ester is chosen from the products corresponding to the formula (II): 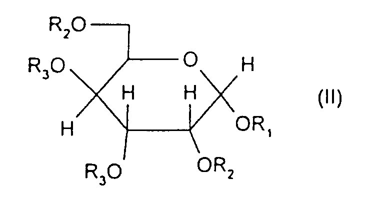 in which R₁ denotes a hydrogen atom or a C₁-C₆ saturated or unsaturated, linear or branched alkyl group, the groups R₂, which are identical or different, denote a proton or a group: 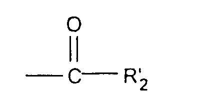 R'₂ denoting a C₁₂-C₂₂ saturated or unsaturated, linear or branched alkyl group, at least one of the groups R₂ being other than a proton, and the groups R₃, which are identical or different, denote a hydrogen atom or a -(CH₂-CH₂O)ₙ-H group, at least one of the groups R₃ being other than a proton, with n ranging from 50 to 200.

9. Composition according to any one of claims 6 to 8, characterized in that it includes up to 15 % by weight of at least one alkylglucose ester.

10. Composition according to any one of the preceding claims, characterized in that it includes a mixture of sugar esters consisting of 30 to 100 % of at least one sucrose ester according to formula (I) and of 0 to 70 % of at least one glucose ester according to formula (II), by weight relative to the total weight of the composition.

11. Composition according to any one of the preceding claims, characterized in that it includes a mixture of sugar esters consisting of 60 to 80 % of at least one sucrose ester according to formula (I) and of 20 to 40 % of at least one glucose ester according to formula (II).

12. Composition according to any one of the preceding claims, characterized in that the film-forming polymer of polyester type additionally includes sequences of amine, amide or urethane type and/or fatty chains.

13. Composition according to any one of the preceding claims, characterized in that the film-forming polymer of polyester type is dispersible in water.

14. Composition according to any one of the preceding claims, characterized in that the film-forming polymer is chosen from water-dispersible anionic polyester and/or polyesteramide polymers including monomers carrying an -SO₃M functional group, M denoting a hydrogen atom, an ammonium ion NH₄⁺ or a metal ion.

15. Composition according to any one of the preceding claims, characterized in that the film-forming polyester is based on at least one dicarboxylic acid, at least one diol and at least one difunctional aromatic monomer additionally carrying an -SO₃M group.

16. Composition according to claim 15, characterized in that the dicarboxylic acid is chosen from phthalic acid, isophthalic acid and terephthalic acid.

17. Composition according to either of claims 15 to 16, characterized in that the diol is chosen from ethylene glycol, diethylene glycol, triethylene glycol, 1,3-propanediol, cyclohexanedimethanol and 4-butanediol.

18. Composition according to any one of claims 15 to 17, characterized in that the difunctional aromatic monomer additionally carrying an -SO₃M group is chosen from sulphoisophthalic acid, sulphoterephthalic acid, sulphophthalic acid and 4-sulphonaphthalene-2,7-dicarboxylic acid.

19. Composition according to any one of the preceding claims, characterized in that the film-forming polymer is based on isophthalate/sulphoisophthalate.

20. Composition according to any one of the preceding claims, characterized in that the film-forming polymer is obtained by condensation of diethylene glycol, cyclohexanedimethanol, isophthalic acid and sulphoisophthalic acid.

21. Composition according to any one of the preceding claims, characterized in that it includes a quantity of film-forming polymer ranging from 0.1 to 25 % by weight relative to the total weight of the composition.

22. Composition according to claim 21, characterized in that it includes a quantity of film-forming polymer ranging from 1 to 10 % by weight relative to the total weight of the composition.

23. Composition according to any one of the preceding claims, characterized in that it additionally includes at least one coemulsifier.

24. Composition according to claim 23, characterized in that the coemulsifier is chosen from fatty alcohols, fatty acids and glycerol fatty esters.

25. Composition according to any one of the preceding claims, characterized in that it includes a quantity of wax ranging from 5 to 30 % by weight relative to the total weight of the composition.

26. Composition according to any one of the preceding claims, characterized in that it is in the form of a wax-in-water emulsion, of an aqueous dispersion, of an oily dispersion, of a water-in-oil emulsion, of an oil-in-water emulsion, of a water-in-wax emulsion or of a gel.

27. Composition according to any one of the preceding claims, characterized in that it is in the form of a make-up product such as mascara, lip rouge, foundation, eyeliner, a hair-styling or hair-care product or of a skin care product such as a care cream or sun product.

28. Use of a composition according to any one of the preceding claims, characterized in that it is employed for preparing a mascara.

29. Use of an ester of fatty acid and of sucrose, in a cosmetic composition including at least one wax and at least one film-forming and anionic polymer of polyester type, as emulsifier.

30. Use of an ester of fatty acid and of sucrose, in a mascara composition including at least one wax and at least one film-forming and anionic polymer of polyester type, for the purpose of improving the lengthening of the eyelash, the curving of the eyelash and/or the adhesion of the mascara to the eyelash.

31. Use according to one of claims 28 to 30, in a mascara composition intended for sensitive eyes.

## Patentansprüche

1. Kosmetische Zusammensetzung, dadurch gekennzeichnet, daß sie enthält:
- mindestens ein Wachs
- mindestens ein anionisches und filmbildendes Polymer vom Polyester-Typ und
- mindestens einen Ester aus einer Fettsäure und Saccharose.

2. Zusammensetzung nach Anspruch 1, dadurch gekennzeichnet, daß sie, angegeben in Gew.-%, bezogen auf das Gesamtgewicht der Zusammensetzung, enthält:
- 5 bis 30 % mindestens eines Wachses,
- 0,1 bis 25 % mindestens eines anionischen und filmbildenden Polymers vom Polyester-Typ und
- 0,5 bis 20 % mindestens eines Esters aus einer Fettsäure und Saccharose.

3. Zusammensetzung nach einem der vorhergehenden Ansprüche, dadurch gekennzeichnet, daß der Veresterungsgrad der Saccharose in dem Ester aus einer Fettsäure und Saccharose größer als oder gleich zwei ist.

4. Zusammensetzung nach einem der vorhergehenden Ansprüche, dadurch gekennzeichnet, daß der Ester aus einer Fettsäure und Saccharose ausgewählt ist unter den Verbindungen der Formel (I), 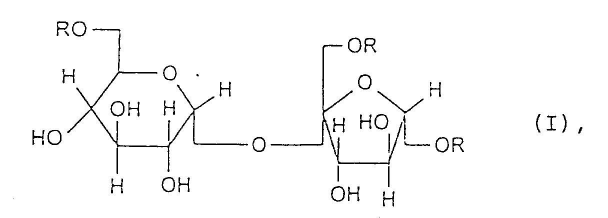 in der die Substituenten R, die gleich oder verschieden sind, ein Wasserstoffatom oder eine Gruppe 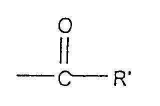 darstellen, worin R' eine geradkettige oder verzweigte, gesättigte oder ungesättigte C₁₂₋₂₂-Alkylgruppe bedeutet, wobei mindestens einer der Substituenten R von Wasserstoff verschieden ist.

5. Zusammensetzung nach einem der vorhergehenden Ansprüche, dadurch gekennzeichnet, daß sie außerdem mindestens einen Monosaccharidester und/oder Alkylmonosaccharidester enthält.

6. Zusammensetzung nach Anspruch 5, dadurch gekennzeichnet, daß der Monosaccharidester und/oder der Alkylmonosaccharidester ein Alkylglucoseester ist.

7. Zusammensetzung nach Anspruch 6, dadurch gekennzeichnet, daß der Alkylglucoseester unter den polyethoxylierten Fettestern von C₁₋₆-Alkylglucose ausgewählt ist.

8. Zusammensetzung nach einem der Ansprüche 6 und 7, dadurch gekennzeichnet, daß der Alkylglucoseester unter den Verbindungen der Formel (II) 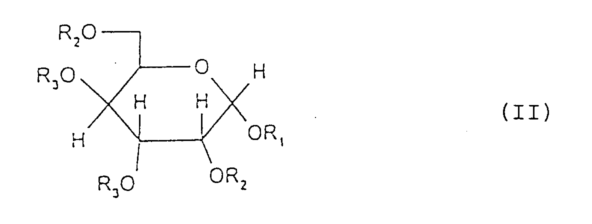 ausgewählt ist, in der bedeuten: R₁ ein Wasserstoffatom oder eine gesättigte oder ungesättigte, geradkettige oder verzweigte C₁₋₆-Alkylgruppe, die Gruppen R₂ ein Wasserstoffatom oder eine Gruppe 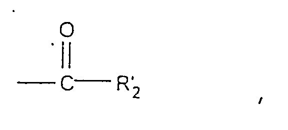 worin R'₂ eine gesättigte oder ungesättigte, geradkettige oder verzweigte C₁₂₋₂₂-Alkylgruppe ist, wobei mindestens eine der Gruppen R₂ von Wasserstoff verschieden ist, und die Gruppen R₃, die gleich oder verschieden sind, ein Wasserstoffatom oder eine Gruppe -(CH₂-CH₂-O)ₙ-H mit n im Bereich von 50 bis 200, wobei mindestens eine der Gruppen R₃ von Wasserstoff verschieden ist.

9. Zusammensetzung nach einem der Ansprüche 6 bis 8, dadurch gekennzeichnet, daß sie bis zu 15 Gew.-% mindestens eines Alkylglucoseesters enthält.

10. Zusammensetzung nach einem der vorhergehenden Ansprüche, dadurch gekennzeichnet, daß sie ein Gemisch von Zuckerestern enthält, das aus 30 bis 100 Gew.-% mindestens eines Saccharoseesters nach Formel (I) und 0 bis 70 Gew.-% mindestens eines Glucoseesters nach Formel (II), bezogen auf das Gesamtgewicht der Zusammensetzung, besteht.

11. Zusammensetzung nach einem der vorhergehenden Ansprüche, dadurch gekennzeichnet, daß sie ein Gemisch von Zuckerestern enthält, das aus 60 bis 80 Gew.-% mindestens eines Saccharoseesters nach Formel (I) und 20 bis 40 Gew.-% mindestens eines Glucoseesters nach Formel (II) besteht.

12. Zusammensetzung nach einem der vorhergehenden Ansprüche, dadurch gekennzeichnet, daß das filmbildende Polymer vom Polyester-Typ außerdem Sequenzen vom Amin-, Amid-, Urethantyp und/oder Fettketten enthält.

13. Zusammensetzung nach einem der vorhergehenden Ansprüche, dadurch gekennzeichnet, daß das filmbildende Polymer vom Polyester-Typ in Wasser dispergierbar ist.

14. Zusammensetzung nach einem der vorhergehenden Ansprüche, dadurch gekennzeichnet, daß das filmbildende Polymer ausgewählt ist unter in Wasser dispergierbaren, anionischen Polyesterpolymeren und/oder Polyesteramiden, die Monomereinheiten enthalten, die eine Gruppe -SO₃M tragen, worin M ein Wasserstoffatom, ein Ammoniumion NH₄⁺ oder ein Metallion bedeutet.

15. Zusammensetzung nach einem der vorhergehenden Ansprüche, dadurch gekennzeichnet, daß der filmbildende Polyester ein Polyester auf der Basis mindestens einer Dicarbonsäure, mindestens eines Diols und mindestens eines aromatischen bifunktionellen Monomers, das außerdem eine Gruppe -SO₃M trägt, ist.

16. Zusammensetzung nach Anspruch 15, dadurch gekennzeichnet, daß die Dicarbonsäure unter Phthalsäure, Isophthalsäure, Terephthalsäure ausgewählt ist.

17. Zusammensetzung nach einem der Ansprüche 15 und 16, dadurch gekennzeichnet, daß das Diol unter Ethylenglykol, Diethylenglykol, Triethylenglykol, 1,3-Propandiol, Cyclohexandimethanol, 4-Butandiol ausgewählt ist.

18. Zusammensetzung nach einem der Ansprüche 15 bis 17, dadurch gekennzeichnet, daß das aromatische bifunktionelle Monomer, das außerdem eine Gruppe -SO₃M trägt, unter Sulfoisophthalsäure, Sulfoterephthalsäure, Sulfophthalsäure, 4-Sulfonaphthalin-2,7-dicarbonsäure ausgewählt ist.

19. Zusammensetzung nach einem der vorhergehenden Ansprüche, dadurch gekennzeichnet, daß das filmbildende Polymer ein Polymer auf Isophthalat/Sulfoisophthalat-Basis ist.

20. Zusammensetzung nach einem der vorhergehenden Ansprüche, dadurch gekennzeichnet, daß das filmbildende Polymer durch Kondensation von Diethylenglykol, Cyclohexandimethanol, Isophthalsäure, Sulfoisophthalsäure hergestellt wird.

21. Zusammensetzung nach einem der vorhergehenden Ansprüche, dadurch gekennzeichnet, daß sie eine Menge an filmbildendem Polymer im Bereich von 0,1 bis 25 Gew.-%, bezogen auf das Gesamtgewicht der Zusammensetzung, enthält.

22. Zusammensetzung nach Anspruch 21, dadurch gekennzeichnet, daß sie eine Menge an filmbildendem Polymer im Bereich von 1 bis 10 Gew.-%, bezogen auf das Gesamtgewicht der Zusammensetzung, enthält.

23. Zusammensetzung nach einem der vorhergehenden Ansprüche, dadurch gekennzeichnet, daß sie außerdem mindestens einen Co-Emulgator enthält.

24. Zusammensetzung nach Anspruch 23, dadurch gekennzeichnet, daß der Co-Emulgator unter Fettalkoholen, Fettsäuren und den Fettestern von Glycerin ausgewählt ist.

25. Zusammensetzung nach einem der vorhergehenden Ansprüche, dadurch gekennzeichnet, daß sie eine Wachsmenge im Bereich von 5 bis 30 Gew.-%, bezogen auf das Gesamtgewicht der Zusammensetzung, enthält.

26. Zusammensetzung nach einem der vorhergehenden Ansprüche, dadurch gekennzeichnet, daß sie in Form einer Wachs-in-Wasser-Emulsion, einer wäßrigen Dispersion, einer öligen Dispersion, einer Wasser-in-Öl-Emulsion, einer Öl-in-Wasser-Emulsion, einer Wasser-in-Wachs-Emulsion oder eines Gels vorliegt.

27. Zusammensetzung nach einem der vorhergehenden Ansprüche, dadurch gekennzeichnet, daß sie in Form eines Schminkprodukts, z.B. Mascara, Lippenstift, Make-up, Eyeliner, in Form eines Frisierprodukts oder Pflegeprodukts für die Haare oder in Form eines Pflegeprodukts für die Haut, wie z.B. einer Pflegecreme oder eines Lichtschutzmittels, vorliegt.

28. Verwendung einer Zusammensetzung nach einem der vorhergehenden Ansprüche, dadurch gekennzeichnet, daß sie für die Herstellung von Mascara verwendet wird.

29. Verwendung eines Esters aus einer Fettsäure und Saccharose als Emulgator in einer kosmetischen Zusammensetzung, die mindestens ein Wachs und mindestens ein anionisches und filmbildendes Polymer vom Polyester-Typ enthält.

30. Verwendung eines Esters aus einer Fettsäure und Saccharose in einer Mascara-Zusammensetzung, die mindestens ein Wachs und mindestens ein anionisches und filmbildendes Polymer vom Polyestertyp enthält, zur Verbesserung der Verlängerung der Wimpern, der Krümmung der Wimpern und/oder der Haftung des Mascaras an den Wimpern.

31. Verwendung nach einem der Ansprüche 28 bis 30 in einer Mascara-Zusammensetzung, die für empfindliche Augen bestimmt ist.
